# EUROPEAN PATENT APPLICATION

(11) **EP 1 437 592 A1**
(43) Date of publication of application: **14.07.2004**
(21) Application number: 02765506.7
(22) Date of filing: 12.09.2002
(51) Int. Cl.: G01N 33/50, G01N 33/15, A61K 45/00, A61P 43/00, A61P 25/28, A61P 25/00, A61P 25/04

(54) **NOVEL SCREENING METHOD USING PROKINETICIN RECEPTOR**

(30) Priority: 20.09.2001 JP 2001287448
(71) Applicant: YAMANOUCHI PHARMACEUTICAL CO. LTD., Tokyo 103-8411 (JP)
(72) Inventor: MATSUMOTO, Shunichiro, c/o Yamanouchi, Tsukuba-shi, Ibaraki305-8585 (JP); TAKASAKI, Jun, c/o Yamanouchi, Tsukuba-shi, Ibaraki 305-8585 (JP); SAITO, Tetsu, c/o Yamanouchi, Tsukuba-shi, Ibaraki 305-8585 (JP); SOGA, Takatoshi, c/o Yamanouchi, Tsukuba-shi, Ibaraki 305-8585 (JP); KAMOHARA, Masazumi, c/o Yamanouchi, Tsukuba-shi, Ibaraki 305-8585 (JP); HIYAMA, Hideki, c/o Yamanouchi, Tsukuba-shi, Ibaraki 305-8585 (JP)
(74) Representative: Hiebl, Inge Elisabeth
(86) International application number: PCT/JP2002/009351
(87) International publication number: WO 2003/027672

(57) **Abstract**

An endogenous receptor for human prokineticins 1 and 2, which do not affect gastrointestinal system contractive action and spermatogenesis action but exert influences upon central nervous system actions, particularly neuronal death inhibitory action, was identified. There is disclosed a method for conveniently screening a useful substance which can control central nervous system actions, for example, a substance useful as an agent for treating dementia and stroke known as neuronal death-accompanying diseases and/or hyperalgesia, by using the aforementioned receptor and/or a cell transformed with the aforementioned receptor.

Also, there are disclosed a tool for screening a neuronal death inhibitor, which is a prokineticin receptor GPRg2, a functionally equivalent variant thereof or a homologous polypeptide thereof for use in the production of a neuronal death inhibitor and/or a hyperalgesia-treating agent, and a tool for screening a neuronal death inhibitor, which is a transformed cell expressing the aforementioned polypeptide, prepared by transforming with an expression vector comprising a polynucleotide coding for the aforementioned polypeptide.

In addition, there is disclosed a pharmaceutical composition for neuronal death inhibition and/or a hyperalgesia treatment, which contains, as the active ingredient, a substance capable of modifying activity of the aforementioned receptor and can be obtained by the aforementioned screening tool or screening method.

SEQUENCE LISTING

<110> Yamanouchi Pharmaceutical Co.,Ltd.

<120> NOVEL SCREENING METHODS USING PROKINETICIN RECEPTORS

<130> Y0226-PCT

<150> JP 2001-287448
<151> 2001-09-20

<160> 10

<170> PatentIn Ver. 2.1

<210> 1
<211> 1155
<212> DNA
<213> Homo sapiens

<220>
<221> CDS
<222> (1)..(1155)

<400> 1
atg gca gcc cag aat gga aac acc agt ttc aca ccc aac ttt aat cca 48

1               5                   10                  15

ccc caa gac cat gcc tcc tcc ctc tcc ttt aac ttc agt tat ggt gat 96


            20                  25                  30

tat gac ctc cct atg gat gag gat gag gac atg acc aag acc cgg acc 144

        35                  40                  45

ttc ttc gca gcc aag atc gtc att ggc att gca ctg gca ggc atc atg 192

    50                  55                  60

ctg gtc tgc ggc atc ggt aac ttt gtc ttt atc gct gcc ctc acc cgc 240

65                  70                  75                  80

tat aag aag ttg cgc aac ctc acc aat ctg ctc att gcc aac ctg gcc 288

                85                  90                  95

atc tcc gac ttc ctg gtg gcc atc atc tgc tgc ccc ttc gag atg gac 336

            100                 105                 110

tac tac gtg gta cgg cag ctc tcc tgg gag cat ggc cac gtg ctc tgt 384

        115                 120                 125

gcc tcc gtc aac tac ctg cgc acc gtc tcc ctc tac gtc tcc acc aat 432

    130                 135                 140

gcc ttg ctg gcc att gcc att gac aga tat ctc gcc atc gtt cac ccc 480


145                 150                 155                 160

ttg aaa cca cgg atg aat tat caa acg gcc tcc ttc ctg atc gcc ttg     528

                165                 170                 175

gtc tgg atg gtg tcc att ctc att gcc atc cca tcg gct tac ttt gca     576

            180                 185                 190

aca gaa acg gtc ctc ttt att gtc aag agc cag gag aag atc ttc tgt     624

        195                 200                 205

ggc cag atc tgg cct gtg gat cag cag ctc tac tac aag tcc tac ttc     672

    210                 215                 220

ctc ttc atc ttt ggt gtc gag ttc gtg ggc cct gtg gtc acc atg acc     720

225                 230                 235                 240

ctg tgc tat gcc agg atc tcc cgg gag ctc tgg ttc aag gca gtc cct     768

                245                 250                 255

ggg ttc cag acg gag cag att cgc aag cgg ctg cgc tgc cgc agg aag     816

            260                 265                 270

acg gtc ctg gtg ctc atg tgc att ctc acg gcc tat gtg ctg tgc tgg 864

        275                 280                 285

gca ccc ttc tac ggt ttc acc atc gtt cgt gac ttc ttc ccc act gtg 912

    290                 295                 300

ttc gtg aag gaa aag cac tac ctc act gcc ttc tac gtg gtc gag tgc 960

305                 310                 315                 320

atc gcc atg agc aac agc atg atc aac acc gtg tgc ttc gtg acg gtc 1008

                325                 330                 335

aag aac aac acc atg aag tac ttc aag aag atg atg ctg ctg cac tgg 1056

            340                 345                 350

cgt ccc tcc cag cgg ggg agc aag tcc agt gct gac ctt gac ctc aga 1104

        355                 360                 365

acc aac ggg gtg ccc acc aca gaa gag gtg gac tgt atc agg ctg aag 1152

    370                 375                 380

tga 1155

385

<210> 2
<211> 384
<212> PRT
<213> Homo sapiens

<400> 2

<210> 3
<211> 38
<212> DNA

<213> Artificial Sequence

<220>
<223> Description of Artificial Sequence:an artificially
      synthesized primer sequence

<400> 3
ctagtctaga atgagaggtg ccacgcgagt ctcaatca                              38

<210> 4
<211> 69
<212> DNA
<213> Artificial Sequence

<220>
<223> Description of Artificial Sequence:an artificially
      synthesized primer sequence

<400> 4
gcggccgcct acttatcgtc gtcatccttg taatcctcga gaaaattgat gttcttcaag 60
tccatggag                                                              69

<210> 5
<211> 38
<212> DNA
<213> Artificial Sequence

<220>
<223> Description of Artificial Sequence:an artificially

synthesized primer sequence

<400> 5
ctagtctaga atgaggagcc tgtgctgcgc cccactcc 38

<210> 6
<211> 69
<212> DNA
<213> Artificial Sequence

<220>
<223> Description of Artificial Sequence:an artificially
synthesized primer sequence

<400> 6
gcggccgcct acttatcgtc gtcatccttg taatcctcga gcttttgggc taaacaaata 60
aatcggtta 69

<210> 7
<211> 38
<212> DNA
<213> Artificial Sequence

<220>
<223> Description of Artificial Sequence:an artificially
synthesized primer sequence

<400> 7
ctagtctaga atggcagccc agaatggaaa caccagtt 38

<210> 8
<211> 38
<212> DNA
<213> Artificial Sequence

<220>
<223> Description of Artificial Sequence:an artificially
      synthesized primer sequence

<400> 8
ctagtctaga ttacttcagc ctgatacagt ccacctct                    38



<210> 9
<211> 236
<212> DNA
<213> Artificial Sequence

<220>
<223> Description of Artificial Sequence:an artificially
      synthesized primer sequence

<400> 9
actagtatga agacgatcat cgccctgagc tacatcttct gcctggtatt cgccgactac 60
aaggacgatg atgacaagtc tagacaccat catcatcatc attcttctgg tctggtgcca 120
cgcggttctg gtatgaaaga aaccgctgct gctaaattcg aacgccagca catggacagc 180
ccagatctgg gtaccggtgg tggctccggt atcgaaggtc gtgctgtgat cacagg     236



<210> 10
<211> 53
<212> DNA

<213> Artificial Sequence

<220>
<223> Description of Artificial Sequence:an artificially
      synthesized primer sequence

<400> 10
actagtggtc ccaggtgggg accctcactc tagattaaaa attgatgttc ttc          53

## Description

### Technical Field

This invention relates to a method for screening a substance capable of controlling neuronal death and/or hyperalgesia, which uses a prokineticin receptor.

### Background of the Invention

Prokineticin is a physiologically active protein which is present in human as two subtypes, prokineticin 1 and prokineticin 2 (*Mol. Pharmacol.,* 2001, 59: 692 - 8). Originally, prokineticin was identified in 1990 by a group in France as a snake venom component of a mamba (a large poisonous snake belonging the cobra family, distributing in South Africa) and named "MIT1" (*Toxicon.,* 1990, 28: 847 - 56). The corresponding molecule was purified later from the skin of a frog by a group in Austria and named "Bv8" (*Eur. J. Pharmacol.,* 1999, 374: 189 - 96). Regarding prokineticin in mammals, a mouse prokineticin 2 gene sequence was reported in 1999 (*FEBS Lett.*, 1999, 26: 177 - 81), and it was confirmed further that this gene is present in the 6th chromosome in mice and in the 3rd chromosome p21 in human (Gene, 2000, 3: 189 - 95). In 2001, the presence of human prokineticin 1 was confirmed for the first time by human genomic sequence data base search (*Mol. Pharmacol.,* 2001, 59: 692 - 8).

Prokineticins 1 and 2 are synthesized in cells as precursors which are constituted by 105 and 108 amino acids, respectively, and it is considered that they become 86 in prokineticin 1 and 81 in prokineticin 2 in mature molecules due to the elimination of signal peptides. Prokineticins 1 and 2 have about 55% of homology each other and also have a homology of 50% or more for the corresponding molecules of the mamba and frog. Five disulfide bonds are present in the molecule, and the positions of cysteins are also conserved in MIT1 and Bv8. The steric structure of prokineticin is markedly stable, and it shows resistance even against a protease treatment.

Based on the tissue expression analysis, prokineticin 1 shows a broad expression pattern in central nervous and peripheral tissues such as the brain, the ovary, the kidney, the uterus, the heart, the testis and the like. On the other hand, a broad tissue expression is also observed in prokineticin 2, but the quantity of its mRNA transcription is low in comparison with prokineticin 1 (FEBS Lett., 1999, 462: 177 - 81, *Mol. Pharmacol.,* 2001, 59: 692 - 8).

Regarding physiological functions of prokineticin, (1) in the central nervous system, neuronal death inhibitory activity (*Eur. J. Neurosci.*, 2001, 13: 1694 - 702) and hyperalgesia-inducing activity (*Eur. J. Pharmacol.,* 1999, 374: 189 - 96), (2) the gastrointestinal system contractive action (*Mol. Pharmacol.,* 2001, 59: 692 - 8, *FEBS Lett.*, 1999, 461: 183 - 8) and (3) spermatogenesis action (*FEBS Lett.*, 1999, 462: 177 - 81) have been reported. The neuronal death inhibitory activity of prokineticin was confirmed by apoptosis-inducing tests in which potassium concentration in a proliferation medium is increased using a rat cerebellar granule primary culture cell. In addition, since the same activity disappeared by PD98059 and LY294002, which are inhibitors for MAP kinase (mitogen-activated kinase, MAPK) and phosphatidylinositol-3-kinase (PI-3-K), it is considered that prokineticin carries out intracellular signal transduction via MAPK/PI-3-K. Regarding the hyperalgesia-inducing activity, since prokineticin does not exert influences upon opioid system-mediated hyperalgesia and upon intercerebral receptor binding of opioid ligand, it is considered that hyperalgesia is induced by a system independent of the opioid system.

Though physiological functions of prokineticin have been reported as described in the above, its endogenous receptor has not been identified until recently, and construction of a convenient screening system for compounds capable of controlling central nervous system actions of prokineticin has not been made. Though a prokineticin receptor is disclosed in WO 01/163609, it is described that said receptor is expressed mainly in testis. Accordingly, great concern has been directed toward the identification of endogenous receptor molecules for human prokineticins 1 and 2, which do not affect gastrointestinal system contractive action and spermatogenesis action but exert influences upon central nervous system actions, particularly neuronal death inhibitory action and/or hyperalgesia-controlling action, as well as a method for screening a neuronal death inhibitor and/or a hyperalgesia-treating agent.

### Disclosure of the Invention

As a result of conducting extensive studies, the present inventors have succeeded in identifying a gene coding for an endogenous receptor, GPRg2, for human prokineticins 1 and 2. The inventors have identified that the GPRg2 gene is expressed in human amygdaloid nucleus, hippocampus, callous body, substantia nigra, thalamus, frontal lobe and fetal brain but not expressed in the stomach, small intestines and testis and also it is expressed in spinal cord dorsal root ganglion in the nerve tissue, which undergoes projection of primary perception nerve and carries a role in transmitting a stimulus of pain sensed by a nociceptor to the brain. Accordingly, the inventors have found that the GPRg2, which is an endogenous receptor molecules for human prokineticins 1 and 2 does not affect gastrointestinal system contractive action and spermatogenesis action but exert influences upon central nervous system actions. Also, the inventors have enabled the expression and purification of GPRg2, and production of transformed cells capable of expressing GPRg2. In addition, by finding that intracellular calcium concentration in GPRg2-expressed cells is changed by prokineticin and that prokineticin specifically binds to a membrane fraction of CHO cell stably expressing GPRg2, it was found that the GPRg2 and a GPRg2-expressing cell can be used as tools for screening a substance capable of controlling neuronal death inhibition and/or a substance capable of controlling hyperalgesia. Based on these, a substance capable of controlling action of for the neuronal death inhibitory action and/or hyperalgesia can be identified by selecting a substance which modifies the activity of prokineticin receptor. And what is more, this will lead to the identification of substances useful for agents to treat diseases generated by neuronal death, such as a dementia-treating agent and stroke-treating agent, and/or a hyperalgesia. The present invention contemplates providing screening tools and screening methods based on these findings.

Accordingly, the present invention relates to
(1) a screening tool for a neuronal death inhibitor, which is a polypeptide consisting of an amino acid sequence of SEQ ID NO:2, or a polypeptide comprising an amino acid sequence represented by SEQ ID NO:2 in which 1 to 10 amino acids therein are deleted, substituted and/or added, wherein
   its activity can be verified based on the increase in intracellular calcium concentration in the presence of prokineticin,
(2) a screening tool for a neuronal death inhibitor, which is a polypeptide consisting of an amino acid sequence having a 90% or more of homology with an amino acid sequence of SEQ ID NO:2, wherein
   its activity can be verified based on the increase in intracellular calcium concentration in the presence of prokineticin,
(3) a screening tool for a dementia-treating agent and a stroke-treating agent as neuronal death inhibitors and/or a hyperalgesia-treating agent, which is the polypeptide described in (1) or (2)
   (hereinafter, the tool for screening a neuronal death inhibitor and the tool for screening a dementia-treating agent, a stroke-treating agent and a hyperalgesia-treating agent are generally referred to as a tool for screening a polypeptide-type neuronal death inhibitor),
(4) a screening tool for a neuronal death inhibitor, which is a cell expressing the polypeptide described in (1) or (2),
(5) a screening tool for a dementia-treating agent and a stroke-treating agent as a neuronal death inhibitor and/or a hyperalgesia-treating agent, which is the cell described in (4)
   (hereinafter, the tool for screening a neuronal death inhibitor and the tool for screening a dementia-treating agent, a stroke-treating agent and a hyperalgesia-treating agent described in (4) and (5) are generally referred to as a tool for screening a transformed cell-type neuronal death inhibitor),
(6) a method for detecting whether or not a compound to be tested is a prokineticin receptor antagonist or an antagonist, comprising the steps of:
   allowing the cell according to (4) or (5), or its cell membrane thereof to contact with a compound to be tested in the presence of prokineticin;
   analyzing changes in the activity of the said polypeptide,
(7) a method for screening a neuronal death inhibitor, comprising the steps of:
   allowing the cell according to (4) or (5), or a cell membrane thereof to contact with a compound to be tested in the presence of prokineticin; and
   analyzing changes in the activity of the said polypeptide,
(8) a method for screening a dementia-treating agent and a stroke-treating agent as neuronal death inhibitors and/or a hyperalgesia-treating agent, comprising the steps of allowing the cell according to (4) or (5), or a cell membrane thereof to contact with a compound to be tested in the presence of prokineticin; and
   analyzing changes in the activity of the said polypeptide, and
(9) a process for producing a pharmaceutical composition for dementia treatment and stroke treatment, or for neuronal death inhibition, and/or hyperalgesia treatment, comprising the steps of carrying out screening using the screening method according to (7) or (8); and producing a pharmaceutical preparation using the substance obtained by the said screening.

The term "screening tool" as used herein means a material to be used for the screening (illustratively, a polypeptide or polypeptide-expressing cell to be used for the screening). The "neuronal death inhibitor-screening tool" is a cell or peptide to which a substance to be tested is contacted in order to screen a neuronal death inhibitor and/or a hyperalgesia-treating agent by the method of the present invention for screening a neuronal death inhibitor and/or a hyperalgesia-treating agent.

The use of the polypeptide or cell described in (1) to (5) for screening a neuronal death inhibitor and/or a hyperalgesia-treating agent is also included in the present invention.

The receptor disclosed in the aforementioned WO 01/163609 as a prokineticin receptor mainly expressing in testis has 87% of homology with the prokineticin receptor GPRg2 which can be used in the present invention. On the other hand, there are various reports regarding a polynucleotide coding for a polypeptide consisting of the same amino acid sequence of the prokineticin receptor, GPRg2, which can be used in the present invention and a deduced amino acid sequence encoded by said polynucleotide (WO 98/46620, WO 01/36471, WO 01/53308, WO 01/68699, WO 01/68700), but its ligand and use are not elucidated. In this connection, a paper published after the priority date of the instant application (JBC 277, 22, pp. 19276 - 19280, 2002) discloses that a protein having the same sequence of the prokineticin receptor which can be used in the present invention is a receptor of prokineticin, and the same paper describes that the aforementioned receptor is concerned in the contraction of the small intestines and angiogenesis. In addition, WO 02/6483 opened after the priority date of the instant application describes about binding of a receptor called I5E identical to the prokineticin receptor GPRg2 with a prokineticin 1 called human ZAQ ligand, but it does not describe that such binding is concerned in the central nervous system actions and it does not describe that the aforementioned receptor binds to prokineticin 2.

As described in the foregoing, the tool for screening a neuronal death inhibitor, the method for screening a neuronal death inhibitor and/or a hyperalgesia-treating agent, and the method for producing a pharmaceutical composition for dementia treatment, stroke treatment and/or hyperalgesia treatment, namely for neuronal death inhibition, described in the instant application are inventions accomplished for the first time by the inventors of the instant application.

### Brief Description of the Drawings

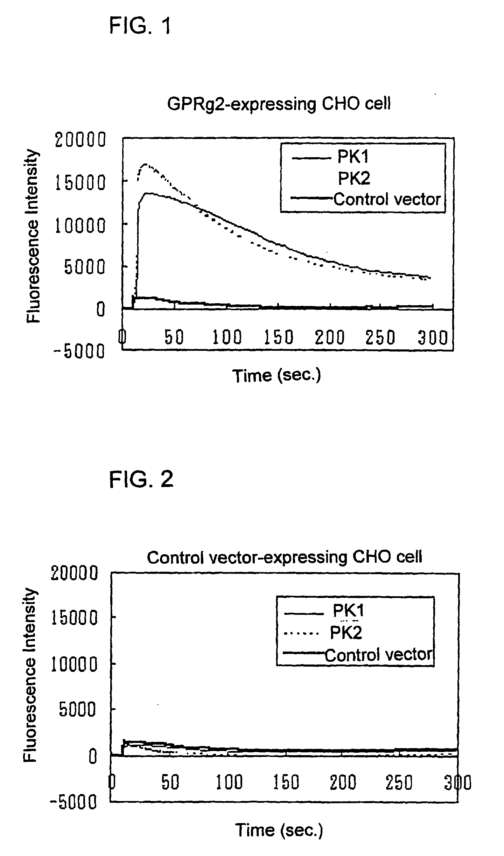

Fig. 1 is a graph showing periodical changes in the calcium concentration when a prokineticin receptor GPRg2-expressed CHO cell was used and an HEK 293 culture supernatant containing prokineticin 1 (PK1) or prokineticin 2 (PK2) was added thereto.
Fig. 2 is a graph showing changes with time in the calcium concentration when a control vector-expressing CHO cell was used and an HEK 293 culture supernatant containing prokineticin 1 (PK1) or prokineticin 2 (PK2) was added thereto.

### Best Mode for Carrying Out the Invention

The following describes the present invention in detail.

The term "prokineticin receptor" as used herein means a "prokineticin receptor protein". The term "prokineticin" includes "prokineticin 1" and "prokineticin 2". The term "agonist" represents a substance which accelerates the activity of prokineticin receptor in the presence or absence of prokineticin.

### [1] Neuronal death inhibitor-screening tool

The neuronal death inhibitor-screening tool of the present invention include a polypeptide-type neuronal death inhibitor-screening tool and a transformed cell-type neuronal death inhibitor-screening tool. In addition, a dementia-treating agent, stroke-treating agent or hyperalgesia-treating agent screening tool is also included in the neuronal death inhibitor-screening tool.

### 1) Polypeptide-type neuronal death inhibitor-screening tool

Examples of the polypeptide which can be used as the polypeptide-type neuronal death inhibitor-screening tool of the present invention include
(1) a polypeptide consisting of an amino acid sequence of SEQ ID NO:2;
(2) a polypeptide comprising an amino acid sequence represented by SEQ ID NO:2 in which from 1 to 10, preferably from 1 to 7, more preferably from 1 to 5 amino acids in the amino acid sequence are deleted, substituted and/or added, wherein its activity can be verified based on the change in intracellular calcium concentration in the presence of prokineticin (preferably prokineticin 2) (to be referred to as "functionally equivalent variant" hereinafter); and
(3) a polypeptide consisting of an amino acid sequence having a 90% or more of homology with an amino acid sequence of SEQ ID NO:2, wherein its activity can be verified based on the change in intracellular calcium concentration in the presence of prokineticin (preferably prokineticin 2) (to be referred to as "homologous polypeptide" hereinafter). In the following, these various polypeptides which can be used as the polypeptide-type neuronal death inhibitor-screening tool of the present invention are generally referred to as polypeptide for screening.

The homologous polypeptide of the present invention is not particularly limited, with the proviso that it is a polypeptide consisting of an amino acid sequence having a 90% or more of homology with an amino acid sequence of SEQ ID NO:2, wherein its activity can be verified based on the change in intracellular calcium concentration in the presence of prokineticin, but it can may comprise an amino acid sequence having preferably 90% or more, more preferably 95% or more, further preferably 98% or more, of homology regarding an amino acid sequence of SEQ ID NO:2. In this connection, the aforementioned term "homology" as used in this specification means a value obtained by a BLAST (basic local alignment search tool; Altschul, S.F. et al., J. Mol. Biol., 215, 403 - 410, 1990) retrieval. The BLAST retrieval can be carried out using b12seq program (Tatiana A. Tatusova, Thomas L. Madden, *FEMS Microbiol.* Lett., 174, 247 - 250, 1999) of a BLAST package [sgi 32 bit edition, version 2.0.12; obtainable from National Center for Biotechnology Information (NCBI)]. Regarding parameters in this case, "blastp" is used as the "program name", and "0" as the "Gap insertion Cost value", "0" as the "Gap elongation Cost value" and "BLOSUM62" as "Matrix", as pair wise alignment parameters.

Thus, the polypeptides for screening use were described in the foregoing, of which a "polypeptide consisting of an amino acid sequence of SEQ ID NO:2" or a "polypeptide consisting of an amino acid sequence represented by SEQ ID NO:2 in which from 1 to 10, preferably from 1 to 7, more preferably from 1 to 5 amino acids in the amino acid sequence are deleted, substituted and/or added, wherein its activity can be verified based on the change in intracellular calcium concentration in the presence of prokineticin" is desirable as said polypeptide, and the "polypeptide consisting of an amino acid sequence of SEQ ID NO:2" is more desirable. The polypeptide for screening use is not particularly limited with the proviso that it is the aforementioned polypeptide, and its origin is not limited to human.

For example, not only a mutant in human of the polypeptide consisting of an amino acid sequence of SEQ ID NO:2 is included but also a functionally equivalent variant derived from an organism other than human (e.g., mouse, rat, hamster or dog) is included. Also included are their natural polypeptides (namely, variants derived from human or functionally equivalent variants derived from an organism other than human), and polypeptides obtained by artificially modifying amino acid sequence of the polypeptide consisting of an amino acid sequence of SEQ ID NO:2 by means of genetic engineering. In this connection, the term "variant" (variation) as used herein means an individual difference found in the same polypeptide of the same species or a difference found in homologous polypeptides among several species.

The mutant in human of the polypeptide consisting of an amino acid sequence of SEQ ID NO:2 or the functionally equivalent variant derived from an organism other than human can be obtained by those skilled in the art based on the information of a nucleotide sequence of a polynucleotide coding for the polypeptide consisting of an amino acid sequence of SEQ ID NO:2 (e.g., the nucleotide sequence represented by SEQ ID NO:1 of the Sequence Listing). In this connection, unless other wise noted, the recombinant DNA techniques can be carried out in accordance with conventionally known methods (e.g., Maniatis, T. et al, "Molecular Cloning - A Laboratory Manual", Cold Spring Harbor Laboratory, NY, 1982).

The polypeptide for screening use which can be used as the polypeptide-type neuronal death inhibitor-screening tool of the present invention can be obtained by various conventionally known methods; for example, it can be prepared by conventionally known genetic engineering techniques using a polynucleotide coding for the protein of interest. More illustratively, it can be prepared by culturing a transformed cell for screening tool use which will be described later (namely, a transformed cell transformed with an expression vector comprising a polynucleotide coding for a polypeptide for screening tool use and expressing the aforementioned polypeptide) under such a condition that the polypeptide for screening tool use can be expressed, and then separating and purifying the protein of interest from the culture by a method generally used for the separation and purification of receptor proteins.

Examples of the polynucleotide coding for a polypeptide for screening tool use include a polynucleotide coding for a polypeptide consisting of an amino acid sequence of SEQ ID NO:2, a polynucleotide coding for a functional variant and a polynucleotide coding for a homologous polypeptide. The term "polynucleotide" as used herein includes both DNA and RNA.

Though the method for producing the polynucleotide coding for a polypeptide for screening tool use is not particularly limited, its examples include (A) a PCR-aided method, (B) a method which uses usual genetic engineering techniques (namely, a method in which a transformant containing the cDNA of interest is selected from transformants transformed with a cDNA library) and (C) a chemical synthesis method. Each of these production methods is described in the following one by one.

### (A) PCR-aided method

A mRNA sample is extracted from a human cell or tissue having the ability to produce the prokineticin receptor of the present invention. Next, two primers sandwiching said receptor mRNA or a part of the mRNA are prepared using this mRNA as the template. By carrying out a reverse transcriptase-polymerase chain reaction (to be referred to as RT-PCR hereinafter), said prokineticin receptor cDNA or a part thereof can be obtained. In addition, by integrating the thus obtained human prokineticin receptor cDNA or a part thereof into an appropriate expression vector, the protein of said receptor can be produced through its expression in a host cell.

Firstly, mRNA molecules including the one coding for said protein are extracted by a conventionally known method from a cell or tissue, such as human brain, having the ability to produce the prokineticin receptor of the present invention. As the extraction method, guanidine thiocyanate hot phenol method, guanidine thiocyanate-guanidine hydrochloride method and the like can be exemplified, but guanidine thiocyanate cesium chloride method can be cited as a preferred example. The cell or tissue having the ability to produce said protein can be specified by the Northern blotting method which uses a gene having a nucleotide sequence coding for said protein, or a part thereof, or the Wstern blotting method which uses an antibody specific for said protein.

Purification of mRNA may be carried out in accordance with a usual method; for example, mRNA can be purified by adsorbing to and eluting from an oligo(dT) cellulose column. In addition, mRNA can be further fractionated by method such as a sucrose density gradient centrifugation or the like. Alternatively, a commercially available mRNA already extracted may be used without carrying out extraction of mRNA.

Next, a single-stranded cDNA is synthesized from the thus purified mRNA by carrying out a reverse transcriptase reaction in the presence of a random primer or oligo(dT) primer. This synthesis can be carried out in the usual way. Alternatively, as is used in Example 1 and Example 2, a commercially available cDNA may be used without synthesizing the cDNA. By subjecting the thus obtained single-stranded cDNA to a polymerase chain reaction (Saiki, R.K. *et al*., (1988), *Science,* 239, 487 - 491; to be referred to as "PCR" hereinafter) using two primers sandwiching a partial region of the gene of interest, the prokineticin receptor DNA of interest is amplified. The thus obtained DNA is fractionated by a agarose gel electrophoresis or the like. As occasion demands, a DNA fragment of interest can also be obtained by digesting the aforementioned DNA with restriction enzymes or the like and ligating the fragments.

### (B) Conventional genetic engineering techniques

Using a mRNA prepared by the aforementioned PCR-aided method as the template, a single-stranded cDNA is synthesized using a reverse transcriptase, and then a double-stranded cDNA is synthesized from this single-stranded cDNA. Examples of the method include an S1 nuclease method (Efstratiadis, A. *et al*., *Cell,* 7, 279 - 288, 1976), a Land method (Land, H. *et al*., *Nucleic Acids Res.,* 9, 2251 - 2266, 1981), an O. Joon Yoo method (Yoo, O.J. *et al., Proc. Natl. Acad. Sci. UAS*, 79, 1049 - 1053, 1983), an Okayama-Berg method (Okayama, H. and Berg, P., *Mol. Cell. Biol.,* 2, 161 - 170, 1982) and the like.

Next, a recombinant plasmid comprising the aforementioned double-stranded cDNA is prepared and introduced into an Escherichia coli strain (e.g., DH5α) to effect transformation, and then transformants are selected using a drug resistance for, e.g., tetracycline or ampicillin as the index. For example, when the host cell is an *E. coli* strain, transformation of the host cell can be carried out by the method of Hanahan (Hanahan, D.J., *Mol. Biol.*, 166, 557 - 580, 1983), namely a method in which the aforementioned recombinant DNA is added to competent cells prepared in the presence of CaCl₂, MgCl₂ or RbCl. In this connection, a phage vector such as a lambda or the like can also be used as the vector in addition to a plasmid.

As the method for selecting a transformant having the cDNA of interest from the transformants obtained in this manner, for example, the following methods can be employed, namely (1) a screening method which uses a synthetic oligonucleotide probe, (2) a screening method which uses a probe prepared by PCR and (3) a method in which the polypeptide of interest is screened by producing it in other animal cells.

By the screening method which uses a synthetic oligonucleotide probe, a transformant having the cDNA of interest can be selected for example by the following procedure.

That is, an oligonucleotide corresponding to the entire portion or a part of the polypeptide of the present invention is synthesized and, using this as the probe (after labeling with ³²P or ³³P), hybridized with a nitrocellulose filter on which DNA samples of transformants are denatured and immobilized, and then the thus obtained positive strains are screened and selected. In this connection, when an oligonucleotide for probe use is synthesized, it can be made into a nucleotide sequence derived using the codon usage or into two or more nucleotide sequences through combinations of deducible nucleotide sequences. In the latter case, their kinds can be reduced by including inosine.

By the screening method which uses a probe prepared by PCR, a transformant having the cDNA of interest can be selected for example by the following procedure.

That is, respective oligonucleotides of a sense primer and antisense primer corresponding to parts of the polypeptide of the present invention is synthesized, and PCR is carried out using the combination thereof to amplify a DNA fragment coding for the entire portion or a part of the polypeptide of interest. As the template DNA to be used in this case, a cDNA synthesized by a reverse transcription reaction from mRNA of a cell capable of producing the polypeptide of the present invention or a genomic DNA can be used. The DNA fragment prepared in this manner is labeled for example with ^{32p} or ³³P, and, using this as the probe, colony hybridization or plaque hybridization is carried out to select a transformant having the cDNA of interest.

By the method in which the polypeptide of interest is screened by producing it in other animal cells, a transformant having the cDNA of interest can be selected for example by the following procedure.

That is, the transformants are cultured to amplify polynucleotide molecules, the polynucleotide molecules are transfected into animal cells, and polypeptides encoded by the polynucleotide molecules are produced on the surface of cells. In this case, either of a plasmid which can replicate by itself contains a transcription promoter region or a plasmid which can be integrated into the chromosome of an animal cell can be used. By detecting the polypeptide of the present invention using an antibody for the polypeptide of the present invention, a transformant having the cDNA of interest is selected from the original transformants.

Regarding the method for collecting the polynucleotide of the present invention from the thus obtained transformant of interest, it can be carried out in accordance with a conventionally known method (e.g., Maniatis, T. et al., "Molecular Cloning - A Laboratory Manual", Cold Spring Harbor Laboratory, NY, 1982). For example, it can be carried out by separating a fraction corresponding to a plasmid DNA from cells and cutting out a cDNA region from the thus obtained plasmid DNA.

### (C) Chemical synthesis method

By the method which uses a chemical synthesis method, the polynucleotide of the present invention can be produced by ligating DNA fragments prepared by a chemical synthesis method. Each of the DNA fragments can be synthesized using a DNA synthesizer [e.g., Oligo 1000M DNA Synthesizer (manufactured by Beckman), 394 DNA/RNA Synthesizer (manufactured by Applied Biosystems) or the like].

In addition, the polynucleotide of the present invention can also be produced based on the information of the polypeptide of the present invention, for example by a conventional method such as chemical synthesis of nucleic acids in accordance with the phosphite triester method (Hunkapiller, M. *et al., Nature*, 10, 105 - 111, 1984) or the like. In this connection, the codons corresponding to desired amino acids are by themselves well known, and their selection is also optional and can be determined for example in accordance with the usual way by taking the codon usage of the host to be used into consideration (Crantham, R. et al., *Nucleic Acids Res*., 9, r43 - r74, 1981). In addition, partial modification of the codons of these nucleotide sequences can be carried out by the site specific mutagenesis (Mark, D.F. *et al., Proc. Natl. Acad.* *Sci. USA,* 81, 5662 - 5666, 1984) or the like making use of primers comprising synthetic oligonucleotides coding for the desired modification.

Sequence determination of the DNA samples obtained by the various methods so fat described can be carried out for example by the Maxam-Gilbert chemical modification method (Maxam, A.M. and Gilbert, W., "Methods in Enzymology", 65, 499 - 559, 1980), the dideoxy nucleotide chain termination method (Messing, J. and Vieira, J., Gene, 19, 269 - 276, 1982) and the like.

The polypeptide for screening tool of the present invention can be obtained by the following method. A host cell (preferably a eucaryote, particularly preferably a CHO cell or 293-EBNA cell) can be transformed by integrating an isolated polynucleotide coding for the polypeptide for screening tool again into an appropriate vector DNA.

By culturing the aforementioned transformed cells, the polypeptide for screening tool produced on the cell surface of the aforementioned cells can be separated and purified by various conventionally known separation methods making use of the physical properties, biochemical properties and the like of the aforementioned polypeptide. Illustrative examples of said method include usual protein precipitant treatment, ultrafiltration, various liquid chromatography techniques, such as molecular sieve chromatography (gel filtration), adsorption chromatography, ion exchange chromatography, affinity chromatography, high performance liquid chromatography (HPLC) and the like, dialysis, combinations thereof and the like, which are carried out after solubilization of a membrane fraction containing the receptor protein. In this connection, the membrane fraction can be obtained in accordance with a conventional method. For example, it can be obtained by culturing cells expressing the prokineticin receptor of the present invention on the surface, suspending them in a buffer, and then homogenizing and centrifuging them. In addition, by solubilizing the prokineticin receptor with a mild solubilizing agent (CHAPS, Triton X-100, digitonin or the like), characteristics of the receptor can be maintained even after the solubilization.

Expression of the polypeptide for screening tool use of the present invention after its in-frame fusion with a marker sequence renders possible confirmation of expression of the polypeptide for screening tool use, confirmation of its intracellular localization, facilitation of its purification and the like. Examples of the marker sequence include FLAG epitope, Hexa-Histidine tag, Hemagglutinin tag, myc epitope and the like. In addition, by inserting a specific sequence which is recognized by proteases such as enterokinase, factor Xa, thrombin and the like between the marker sequence and prokineticin receptor, it is possible to cut out and remove the marker sequence moiety with these proteases. For example, there is a report stating that a muscarine acetylcholine receptor and Hexa-Histidine tag were connected using a thrombin recognizing sequence (Hayashi, M.K. and Haga, T., (1996), *J. Biochem.,* 120, 1232 - 1238).

### 2) Transformed cell-type neuronal death inhibitor-screening tool

Examples of the transformed cell which can be used as the transformed cell-type neuronal death inhibitor-screening tool of the present invention (to be referred to as transformed cell for screening tool hereinafter) include
(1) a transformed cell which is transformed with an expression vector comprising a polynucleotide coding for a polypeptide consisting of an amino acid sequence of SEQ ID NO:2 and is expressing the aforementioned polypeptide;
(2) a transformed cell which is transformed with an expression vector comprising a polynucleotide coding for a functionally equivalent variant and is expressing the aforementioned polypeptide; and
(3) a transformed cell which is transformed with an expression vector comprising a polynucleotide coding for a homologous polypeptide and is expressing the aforementioned polypeptide.

The transformed cell for screening tool can be obtained by transforming a host cell (preferably a eucaryote, particularly preferably a CHO cell or 293-EBNA cell) through the integration of a polynucleotide isolated by the aforementioned method, coding for a polypeptide for screening tool, again into an appropriate vector DNA. In addition, it is possible to effect expression of the polynucleotide in respective host cell by introducing an appropriate promoter and a sequence concerned in the gene expression into such a vector.

The aforementioned expression vector is not particularly limited, with the proviso that it comprises a polynucleotide coding for a polypeptide for screening tool, and an example thereof is an expression vector obtained by inserting the aforementioned polynucleotide into a conventionally known expression vector optionally selected in response to the host cell to be used. In addition, the transformed cell for screening tool which can be used as a transformed cell-type neuronal death inhibitor-screening tool is not particularly limited, with the proviso that it is transformed with the aforementioned expression vector, comprises a polynucleotide coding for a polypeptide for screening tool and expresses the aforementioned polypeptide when used as the transformed cell-type neuronal death inhibitor-screening tool; for example, it may be a cell in which the polynucleotide coding for a polypeptide for screening tool is integrated into the chromosome of a host cell, or it may be a cell which contains the polynucleotide coding for a polypeptide for screening tool in the form of an expression vector comprising the same. The transformed cell for screening tool can be obtained, for example, by transforming a desired host cell with an expression vector comprising a polynucleotide coding for a polypeptide for screening tool.

For example, cells of vertebrate, insect, yeast and the like are included in the eucaryote host cell, and a COS cell which is a simian cell (Gluzman, Y. (1981), *Cell,* 23, 175 - 182), a dihydrofolate reductase-deficient strain of Chinese hamster ovary cell (CHO) (Urlaub, G. and Chasin, L.A. (1980), *Proc. Natl. Acad. Sci. USA*, 77, 4216 - 4220), a human fetal kidney HEK293 cell and a 293-EBNA cell (manufactured by Invitrogen) prepared by introducing the Epstein Barr virus EBNA-1 gene into the same cell are frequently used as the vertebral cells, though not limited thereto.

As the expression vector for vertebral cells, those which have a promoter generally positioned in the upstream of a gene to be expressed, an RNA splicing site, a polyadenylation site, a transcription termination sequence and the like can be used, and it may further have a replication origin as occasion demands. Though not particularly limited, examples of said expression vector include pSV2dhfr having SV40 early promoter (Subramani, S. *et al.* (1981), *Mol. Cell. Biol.*, 1, 854 - 864), pEF-BOS having human elongation factor promoter (Mizushima, S. and Nagata, S. (1990), *Nucleic Acids Res.,* 18, 5322), pCEP4 having cytomegalovirus promoter (manufactured by Invitrogen) and the like.

In an example of a case in which COS cell is used as the host cell, those which have the SV40 replication origin, can perform autonomous growth in COS cell, and further have a transcription promoter, a transcription termination signal and an RNA splicing site can be used as the expression vector, and its examples include pME18S (Maruyama, K. and Takebe, Y. (1990), *Med. Immunol.,* 20, 27 - 32), pEF-BOS (Mizushima, S. and Nagata, S. (1990), *Nucleic Acids Res*., 18, 5322), pCDM8 (Seed, B. (1987), Nature, 329, 840 - 842) and the like. Said expression vector can be incorporated into COS cell by the DEAE-dextran method (Luthman, H. and Magnusson, G. (1983), *Nucleic Acids Res*., 11, 1295 - 1308),the calcium phosphate-DNA coprecipitation method (Graham, F.L. and van der Ed, A.J. (1973), *Virology,* 52, 456 - 457), a method which uses FuGENE6 (manufactured by Boehringer Mannheim), or the electroporation method (Neumann, E. et al. (1982), *EMBO J.,* 1, 841 - 845), and a desired transformed cell can be obtained in this way.

Also, when CHO cell is used as the host cell, a transformed cell capable of stably producing a polypeptide for screening use can be obtained by co-transfecting a vector which can express a neo gene functioning as a G418 resistance marker, such as pRSVneo (Sambrook, J. et al. (1989): "Molecular Cloning - A Laboratory Manual", Cold Spring Harbor Laboratory, NY), pSV2-neo (Southern, P.J. and Berg, P. (1982), *J. Mol. Appl. Genet.*, 1, 327 - 341) or the like, together with an expression vector, and selecting a G418-resistant colony. In addition, when 293-EBNA cell is used as the host cell, a desired transformed cell can be obtained using an expression vector which has the Epstein Barr virus replication origin and can perform autonomous growth in the 293-EBNA cell, such as pCEP4 (manufactured by Invitrogen) or the like.

The transformed cell of interest obtained in the above can be cultured in accordance with a conventional method, and the polypeptide for screening use of the present invention is produced inside the cell or on the cell surface. The medium to be used in said culturing can be optionally selected from various conventional media in response to the employed host cell. In the case of the aforementioned COS cell, for example, a medium such as RPMI-1640 medium, Dulbecco's Modified Eagle's minimum essential medium (DMEM) and the like, which is further supplemented with a serum component (such as fetal bovine serum (FBS) or the like) as occasion demands may be used. In the case of the aforementioned 293-EBNA cell on the other hand, Dulbecco's Modified Eagle's minimum essential medium (DMEM) supplemented with a serum component such as fetal bovine serum (FBS) or the like may be used by further supplementing with G418.

### [2] Method for screening a neuronal death inhibitor and/or a hyperalgesia-treating agent

When a polypeptide for screening tool or a transformed cell for screening tool is used, substances (compounds, peptides and antibodies) capable of modifying activity of the polypeptide for screening tool can be detected and screened.

The detection method of the present invention is carried out by measuring changes in the activity of a polypeptide for screening use using an index of activity corresponding to the physiological characteristics of a receptor protein to be used in the screening. The activity to be used as the index is, for example, the binding activity with a ligand or the response for a stimulus caused by the binding of ligand. Illustratively, the detection method described in the following can be exemplified. As the polypeptide for screening use, a cell expressing said polypeptide, a membrane fraction of said cell, a purified product of a protein comprising said polypeptide and the like can also be used. Also, as the compound to be tested for the screening method of the present invention, commercially available compounds, various conventionally known compounds and peptides registered in chemical files, a group of compounds obtained by the combinatorial chemistry techniques (Terrett, N.K. et al. (1995), *Tetrahedron,* 51, 8135 - 8137) and a group of random peptides prepared by applying the phage display method (Felici, F. et al. (1991), *J. Mol. Biol.,* 222, 301 - 310) and the like can be used. In addition, microbial culture supernatants, natural components derived from plants or marine organisms, animal tissue extracts and the like can also become the subject of the screening. Alternatively, a compound or peptide obtained by chemically or biologically modifying a compound or peptide selected by the screening method of the present invention can be used though not limited thereto.

### a) A screening method making use of a ligand binding assay method

The substances (compounds, peptides and antibodies) which bind to the polypeptide for screening use of the present invention can be screened by a ligand binding assay method. A cell membrane expressing said polypeptide for screening use or a purified product of a protein comprising said polypeptide for screening use is prepared. Assay conditions such as buffer solution, ions, pH and the like are optimized, and a cell membrane expressing the same polypeptide for screening use or a purified product of a protein comprising said polypeptide for screening use is incubated in the optimized buffer for a predetermined period of time together with a labeled ligand such as ¹²⁵I-labeled prokineticin (preferably ¹²⁵I-labeled prokineticin 2) and with an agent to be tested. After the reaction, this is filtered using a glass filter or the like and washed with an adequate amount of the buffer, and then the radioactivity remaining on the filter is measured using a γ counter or the like. Using binding inhibition of the thus obtained radioactive ligand as the index, substances (compounds, peptides and antibodies) having agonist activity of said polypeptide for screening use or substances (compounds, peptides and antibodies) having antagonist activity can be screened. The substances having antagonist activity of said polypeptide for screening use, selected by the screening method of the present invention, are useful for example for the treatment and prevention of hyperalgesia. Also, the substances having agonist activity of said polypeptide for screening use, selected by the screening method of the present invention, are useful in inhibiting neuronal death, for example, in treating and preventing dementia and stroke.

The screening method of the present invention can be carried out by a screening method making use of the ligand binding assay method described in WO 01/19986. However, the ligand LTC₄ is replaced by prokineticin, and the receptor LTC₄ receptor by prokineticin receptor (namely the polypeptide for screening use). More illustratively, this is carried out by the method described in Example 7.

### b) A screening method making use of a GTPγS binding method

The substances (compounds, peptides and antibodies) which modify activity of the polypeptide for screening use of the present invention can be screened by a GTPγS binding method (Lazareno, S. and Birdsall, N.J.M. (1993), *Br. J.* Pharmacol., 109, 1120 - 1127). A cell membrane expressing a polypeptide for screening use is mixed with 400 pM of GTPγS labeled with ³⁵S in a solution of 20 mM HEPES (pH 7.4), 100 mM NaCl, 10 mM MgCl₂ and 50 mM GDP. After incubation in the presence or absence of an agent to be tested, this is filtered using a glass filter or the like and then the radioactivity of bonded GTPγS is measured using a liquid scintillation counter or the like. Using increase in the specific GTPγS binding in the presence of an agent to be tested as the index, substances (compounds, peptides and antibodies) having the agonist activity of said polypeptide for screening use can be screened. Also, using inhibition of the increase of GTPγS binding by prokineticin (preferably prokineticin 2) in the presence of an agent to be tested as the index, substances (compounds, peptides and antibodies) having the antagonist activity of said polypeptide for screening use can be screened.

### c) A screening method making use of a change in intracellular Ca²⁺ concentration

The substances (compounds, peptides and antibodies) which modify activity of the polypeptide for screening use of the present invention can be screened making use of a change in the intracellular Ca²⁺ concentration of a cell expressing the polypeptide for screening use. Measurement of the intracellular Ca²⁺ concentration can be carried out using fura2, fluo3 and the like.

Alternatively, it is possible to measure the Ca²⁺ concentration indirectly, by detecting transcription activity of a gene whose transcription quantity is regulated depending on the Ca²⁺ concentration. Illustratively, the Ca²⁺ concentration can be measured by the following method. Firstly, a serum responsive element-linked reporter gene is introduced into a cell which expressed the polypeptide for screening use, and an agent to be tested is added to a culture medium of said cell. Any optional gene capable of forming a detectably signal can be used as the reporter gene. For example, a luciferase gene is desirable as the reporter gene. After incubation at 37°C for 4 hours, the medium is discarded and the cells are lysed to measure the luciferase activity. Using induction of the luciferase activity at the time of the addition of the agent to be tested as the index, substances (compounds, peptides and antibodies) having the agonist activity of the polypeptide for screening use can be screened. Also, after the addition of an agent to be tested to the culture medium of said cell, prokineticin (preferably prokineticin 2) is added thereto to a final concentration of from 5 to 500 nM and the luciferase activity is measured in the same manner. Using inhibition of the luciferase activity induction by prokineticin (preferably prokineticin 2) at the time of the addition of the agent to be tested as the index, substances (compounds, peptides and antibodies) having the antagonist activity of the polypeptide for screening use can be screened.

According to the screening method of the present invention, the Ca²⁺ concentration is measured directly or indirectly by allowing substances (compounds, peptides and antibodies) and the like to react for a predetermined period of time with a cell expressing said protein or a unexpressed host cell (control cell). Using increase or decrease of the Ca²⁺ concentration specific for the cell expressing said protein, in comparison with the control cell, as the index, substances (compounds, peptides and antibodies) having the agonist activity can be screened. Also, using the action to inhibit increase or decrease of Ca²⁺ concentration by prokineticin (preferably prokineticin 2) in the presence of the agent to be tested as the index, substances (compounds, peptides and antibodies) having the antagonist activity of said polypeptide for screening use can be screened.

It is desirable to carry out the screening method of the present invention under the conditions described in Example 4.

For example, under the conditions described in Example 4, a substance of EC₅₀ = 100 µM or less, preferable a substance of EC₅₀ = 10 µM or less, more preferably a substance of EC₅₀ = 1 µM or less, can be selected as a substance having the agonist activity. Also, by adding an agent to be tested under the assay conditions described in Example 4, namely a substance showing an IC₅₀ value of 10 µM or less, preferable a substance showing an IC₅₀ value of 1 µM or less, more preferably a substance showing an IC₅₀ value of 0.1 µM or less, under the conditions described in Example 4 can be selected as a substance having the antagonist activity.

### [3] Method for producing a pharmaceutical composition for neuronal death inhibition and/or hyperalgesia treatment

A method for producing a pharmaceutical composition for neuronal death inhibition and/or hyperalgesia treatment, which comprises a step of carrying out screening using the screening method of the present invention and a step of producing a pharmaceutical preparation using the substance obtained by the aforementioned screening, is included in the present invention.

The pharmaceutical preparations which contain substances obtained by the screening method of the present invention as the active ingredient are prepared using generally used pharmaceutical carriers, excipients and/or other additives in response to the type pf the aforementioned active ingredient.

Examples of the administration include oral administration by tablets, pills, capsules, granules, fine subtilaes, powders, solutions for oral use and the like, and parenteral administration by injections (intravenous, intramuscular, intraarticular and the like), suppositories, percutaneous preparations, transmucosal preparations and the like. Parenteral administration such as intravenous injection or the like is desirable particularly in the case of peptides which are digested in the stomach.

In the solid composition for use in the oral administration, one or more active substances are mixed with at least one inert diluent such as lactose, mannitol, glucose, microcrystalline cellulose, hydroxypropylcellulose, starch, poly(vinyl pyrrolidone), aluminum magnesium silicate or the like. In the usual way, the aforementioned composition may contain other additives than the inert diluent, such as a lubricant, a disintegrating agent, a stabilizing agent and a solubilizing or solubilization assisting agent. If necessary, tablets or pills may be coated with a sugar coating or a film of a gastric or enteric substance.

The liquid composition for oral administration can include, for example, emulsions, solutions, suspensions, syrups or elixirs, and can contain a generally used inert diluent such as purified water or ethanol. In addition to the inert diluent, the aforementioned composition can contain other additive agents such as a moistening agent, a suspending agent, sweeteners, aromatics and antiseptics.

The injections for parenteral administration can include aseptic aqueous or non-aqueous solutions, suspensions or emulsions. Examples of the diluent for use in the aqueous solutions and suspensions include distilled water for injection and physiological saline. Examples of the diluent for use in the non-aqueous solutions or suspensions include propylene glycol, polyethylene glycol, plant oil (e.g., olive oil), alcohols (e.g., ethanol), or polysorbate 80 and the like. The aforementioned composition may further contain a moistening agent, an emulsifying agent, a dispersing agent, a stabilizing agent, a solubilizing or solubilization assisting agent, an antiseptic and the like. The aforementioned compositions can be sterilized, for example, by filtration through a bacteria retaining filter, blending of a germicide or irradiation. Alternatively, they may be used by firstly making into sterile solid compositions and dissolving them in sterile water or other sterile solvent for injection use prior to their use.

The clinical dose can be optionally decided by taking into consideration strength of the activity of the active ingredient, namely that of a substance which inhibits activation of the LTRPC2 protein or a substance obtained by the screening method of the present invention, and symptoms, weight, age, sex and the like of each patient to be treated.

For example, the dose is usually from about 0.1 to about 100 mg, preferably from 0.1 to 50 mg, per day per adult (as 60 kg body weight) in the case of oral administration. In the case of parenteral administration by injections, it is from 0.01 to 50 mg, preferably from 0.01 to 10 mg, per day.

### Examples

The present invention is described in detail in the following examples, but the present invention is not restricted by said examples. In this connection, unless otherwise noted, the experiments can be carried out in accordance with conventionally known methods (e.g., Maniatis, T. et al. (1982): "Molecular Cloning - A Laboratory Manual", Cold Spring Harbor Laboratory, NY). Also, when commercially available reagents and kits are used, they can be carried out in accordance with the instructions of the commercial products.

### (Example 1) Construction of prokineticin expression system in animal cell

In the amplification of genes coding for human prokineticins 1 and 2, a human small intestine cDNA was used as the template in the case of prokineticin 1, or a human testis cDNA in the case of prokineticin 2 (Marathon Ready cDNA; manufactured by Clontech). In the case of prokineticin 1, an oligonucleotide comprising the nucleotide sequence represented by SEQ ID NO:3 was used as the forward primer, and an oligonucleotide comprising the nucleotide sequence represented by SEQ ID NO:4 was used as the reverse primer. In the case of prokineticin 2, an oligonucleotide comprising the nucleotide sequence represented by SEQ ID NO:5 was used as the forward primer, and an oligonucleotide comprising the nucleotide sequence represented by SEQ ID NO:6 was used as the reverse primer. An FLAG sequence is contained in the nucleotide sequences represented by SEQ ID NO:4 and SEQ ID NO:6. Accordingly, the prokineticin is expressed by in-frame fusion of a marker sequence FLAG epitome at the C-terminal side. Based on this, purification of prokineticin can be simplified. In this connection, an XbaI recognizing sequence and a NotI recognizing sequence are respectively added to the 5'-termini of the aforementioned primers. The PCR was carried out using a DNA polymerase (Pyrobest DNA polymerase; manufactured by Takara Shuzo) and, after heating at 94°C (2 minutes), by repeating a cycle of 96°C (5 seconds)/72°C (1.5 minutes) 5 times, a cycle of 96°C (5 seconds)/70°C (1.5 minutes) 5 times, and a cycle of 96°C (5 seconds)/68°C (1.5 minutes) 20 times. As a result, a DNA fragment of about 0.3 kbp was amplified in both cases of prokineticins 1 and 2. Each of these fragments was cloned using pCR2.1 plasmid (manufactured by Invitrogen). Nucleotide sequences of the thus obtained clones were analyzed by the dideoxy terminator method using ABI3700 DNA Sequencer (manufactured by Applied Biosystems), and clones coincided with the known sequences of prokineticins 1 and 2 (GenBank No. AF333024 and AF333025) were selected, respectively. These clones were digested with xbaI and NotI and inserted into a pCEP4 plasmid for animal cell expression (manufactured by Invitrogen).

Regarding the thus constructed prokineticin expression plasmids, the HEK293 cell was inoculated into a 6 well plate (Collagen-Type I-Coated 6 well plate; manufactured by Asahi Technoglass) at a density of 1 x 10⁵ cells per well and cultured for 24 hours, and then 1 µg/well of prokineticin 1 or prokineticin 2 expression plasmid was transfected using a transfection reagent (FuGENE6; manufactured by Boehringer-Mannheim). After 72 hours of the transfection, selection of the transformed cells was carried out using a hygromycin B-containing medium, and the thus obtained drug-resistant cells were used as prokineticin expressing cells. The prokineticin expressing cells were cultured using a 10 cm dish (Collagen-Type 1-Coated; manufactured by Asahi Technoglass) until they became confluent and then, after discarding the medium, cultured for 4 days using DMEM medium containing 0.5% of fetal bovine serum to recover the prokineticin-containing medium.

### (Example 2) Gene cloning of a G protein conjugate type receptor, GPRg2, and preparation of GPRg2-expressing CHO cell

In the amplification of a gene coding for human GPRg2, a human spleen cDNA (Marathon Ready cDNA; manufactured by Clontech) was used as the template, an oligonucleotide comprising the nucleotide sequence represented by SEQ ID NO:7 was used as the forward primer, and an oligonucleotide comprising the nucleotide sequence represented by SEQ ID NO:8 was used as the reverse primer. In this connection, an XbaI recognizing sequence is respectively added to the 5'-termini of the aforementioned primers. The PCR was carried out using a DNA polymerase (Pyrobest DNA polymerase; manufactured by Takara Shuzo) and, after heating at 94°C (2 minutes), by repeating a cycle of 96°C (5 seconds)/72°C (1.5 minutes) 5 times, a cycle of 96°C (5 seconds)/70°C (1.5 minutes) 5 times, and a cycle of 96°C (5 seconds)/68°C (1.5 minutes) 20 times. As a result, a DNA fragment of about 1.2 kbp was amplified. This fragment was digested with XbaI and then inserted into a pEF-BOS-dhfr plasmid (Biochim. Biophys. Acta, 1997, 1354: 159 - 70). Nucleotide sequence of the thus obtained clone was analyzed by the dideoxy terminator method using ABI3700 DNA Sequencer (manufactured by Applied Biosystems). The thus revealed sequence is shown in SEQ ID NO:1.

This sequence has an open reading frame of 1,155 bases (SEQ ID NO: 1). An amino acid sequence (384 amino acids) deduced from the open reading frame is shown in SEQ ID NO:2.

Regarding the thus constructed GPRg2 expression plasmid, the CHO/dhfr⁻ cell was inoculated into a 6 well plate at a density of 1 x 10⁵ cells per well and cultured for 24 hours, and then 1 µg/well of GPRg2 expression plasmid was transfected using a transfection reagent (FuGENE6; manufactured by Boehringer-Mannheim). After 72 hours of the transfection, selection of the transformed cells was carried out using a methotrexate-containing medium, and the thus obtained drug-resistant cells were used as GPRg2 expressing cells.

### (Example 3) Expression analysis of GPRg2 in human tissues

In order to analyze expression of GPRg2 in tissues, PCR was carried out using cDNA preparations derived from various human tissues as the template and using a Taq polymerase (Ex Taq; manufactured by Takara Shuzo). The PCR conditions are identical to described in Example 1. As a result, a DNA fragment of about 1.2 kbp was amplified from the cDNA preparations derived from amygdaloid nucleus, hippocampus, callous body, substantia nigra, thalamus, frontal lobe and fetal brain. The amplification was not found from the cDNA preparations derived from the stomach, small intestines and testis.

### (Example 4) Changes in intracellular Ca²⁺ concentration in GPRg2 expressing CHO cell by prokineticin

A GPRg2 expressing CHO cell or a pEF-BOS-dhfr-integrated CHO cell (control vector-treated cell) was inoculated into a 96 well plate (96 well Black/clear bottom plate; manufactured by BECTON DICKINSON) at a density of 1 x 10⁴ cells and cultured for 24 hours and then, after discarding the medium, incubated at 37°C for 1 hour by adding 100 µl per well of Hanks' balanced salt solution (Hanks BSS; manufactured by Gibco) containing 4 µM Fluo-3, AM (manufactured by Molecular Probe), 0.004% pluronic acid, 1% FBS and 20 mM HEPES. After the incubation, he cells were washed four times with Hanks BSS containing 20 mM HEPES, and then 100 µl per well of Hanks BSS containing 20 mM HEPES was added.

Changes in the intracellular Ca²⁺ concentration were periodically measured using FLIPR (manufactured by Molecular Device). That is, an HEK293 cell culture supernatant containing prokineticin was added 10 seconds after starting the measurement, and fluorescence intensity was measured at an interval of 1 second during 50 seconds after the prokineticin addition and at an interval of 6 seconds during subsequent 4 minutes. When the thus obtained fluorescence value was plotted on the axis Y and the time on the axis X, a change in the intracellular Ca²⁺ concentration was observed due to the action of GPRg2 upon prokineticin, but was not observed in the HEK293 cell culture supernatant in which prokineticin was not expressed. In addition, this reaction was not found in the control vector-treated CHO cell. Based on the above results, it was able to confirmed that GPRg2 is a receptor for prokineticins 1 and 2 in the living body.

It became possible to carry out screening of prokineticin agonist and antagonist, namely a neuronal death inhibitor and/or a hyperalgesia-treating agent, by measuring changes in the intracellular Ca²⁺ concentration in cells transformed with the prokineticin receptor.

### (Example 5) Expression and purification of prokineticins 1 and 2 expressed in animal cell

In order to use a purified prokineticin expressed in an animal cell to a binding assay and the like by labeled with radioisotope, purified prokineticin was firstly obtained by the following experiment. In this case, in order to remove the FLAG sequence used at the time of purification, a prokineticin 1 identical to its native form was prepared by inserting a Factor Xa sequence. Also, since the molecule of prokineticin 2 does not have a Tyr residue to be used for the radioisotope labeling, the Tyr residue in the C-terminus FLAG sequence was attempted to be labeled with the radioisotope. Illustratively, purified prokineticins were obtained by the following procedure.

In order to obtain prokineticin 1 expressed in an animal cell, an oligonucleotide represented by SEQ ID NO:9 was used as the forward primer, and an oligonucleotide represented by SEQ ID NO:10 as the reverse primer. A SpeI recognizing sequence is added to each of the 5'-termini of the aforementioned primers. In this connection, the nucleotide sequence represented by SEQ ID NO:9 contains a signal sequence of influenza A virus hemagglutinin, the FLAG sequence and a Factor Xa recognizing sequence. Accordingly, after its secretion into the extracellular moiety, the prokineticin 1 is expressed as its in-frame fusion with the marker sequence FLAG epitope and Factor Xa recognizing sequence on the N-terminal side. Based on this, purification of prokineticin 1 can be simplified. The PCR was carried out using a Pyrobest DNA polymerase (manufactured by Takara Shuzo) and, after heating at 94°C (2 minutes), by repeating a cycle of 94°C (30 seconds)/55°C (30 seconds)/72°C (1.5 minutes) 20 times. As a result, a DNA fragment of about 1.4 kbp was amplified. This fragment was digested with SpeI and then inserted into the pCEP4 plasmid. Nucleotide sequence of the thus obtained clone was analyzed using ABI3700 DNA Sequencer. A cell strain stably expressing prokineticin 1 was obtained by carrying out gene transfer of the thus constructed prokineticin 1 expressing plasmid into HEK293 cell by the same method used in Example 1.

In order to obtain prokineticin 2 expressed in an animal cell, the cDNA coding for prokineticin 2 expressed as its in-frame fusion with the marker sequence FLAG epitope on the C-terminal side, prepared in Example 1, was used by inserting it into the plasmid pEF-BOS-dhfr. Accordingly, after its secretion into the extracellular moiety, the prokineticin 2 is expressed as its in-frame fusion with the marker sequence FLAG epitope on the C-terminal side. A cell strain stably expressing prokineticin 2 was obtained by carrying out gene transfer of the thus constructed prokineticin 2-expressing plasmid into CHO/dhfr⁻ cell by the same method used in Example 2.

A medium containing prokineticin 1 or prokineticin 2 recovered by the same method of Example 1 was respectively applied to an M2-agarose column (manufactured By Sigma), the column was washed with PBS containing 0.5% NaCl, and then prokineticin 1 and prokineticin 2 were respectively eluted from the M2-agarose column; the former by reacting overnight with Factor Xa (manufactured by Amersham)-containing PBS and the latter with FLAG-peptide-containing PBS. The elution products containing the prokineticins of interest were finally purified by carrying out a gel filtration using a Superdex Peptide FPLC column (manufactured by Amersham). When the purified products were subjected to an SDS electrophoresis and then to a silver staining (manufactured by Daiichi Pure Chemicals), both of prokineticin 1 and prokineticin 2 were verified as a single band of about 10 kDa, the latter being slightly higher molecular weight side than the former. Since the estimated molecular weights of prokineticins 1 and 2 were 9,666.88 Da and 10,039.31 Da, respectively, they coincided with the predicted molecular weights, so that it was able to confirm that prokineticins 1 and 2 were purified.

### (Example 6) Construction of luciferase reporter assay system in GPRg2 expressing HEK293 cell by prokineticin

An HEK293-EBNA cell (manufactured by Invitrogen) was inoculated into a 24 well plate coated with collagen type I (Collagen-Type 1-Coated 24 well plate; manufactured by Asahi Technoglass) to a density of 7 x 10⁴ cells per well and cultured for 24 hours, and then both of the GPRg2 animal cell expressing plasmid constructed in Example 1 or the plasmid pEF-BOS (empty vector as a control) (100 ng per well) and a plasmid pSRE-luc (manufactured by Stratagene) (20 ng per well) were simultaneously subjected to transfection using a gene transfer reagent (FuGENE6; manufactured by Boehringer Mannheim). After a lapse of 24 hours from the transfection, the medium was discarded, 10 nM of the purified prokineticin 1 or purified prokineticin 2 was added thereto and allowed to undergo the reaction at 37°C for 5 hours to measure the intracellular luciferase activity in accordance with the method of Luciferase Assay System (manufactured by Wako Pure Chemical Industries). As a result, the intracellular luciferase activity was specifically increased in the HEK293 cell gene-transfected with GPRg2, in comparison with the HEK293 cell gene-transfected with empty vector.

The use of this reaction system by adding an agent to be tested instead of prokineticin or simultaneously with prokineticin enabled the screening of a substance having an agonist activity exerting similar activity of prokineticin 1 or 2 (namely a neuronal death inhibitor) and of a substance having an antagonist activity of inhibiting the action of prokineticin 1 or 2 upon GPRg2 (namely a hyperalgesia-treating agent).

### (Example 7) Construction of GPRg2 receptor binding assay system using ¹²⁵I-labeled prokineticin 2

Each of a CHO cell and a GPRg2-stably expressing CHO cell was cultured in a 150 mm culture dish until it became confluent, and then respectively suspended in 50 mM Tris-HCl (pH 7.5) containing 10 mM MgCl₂ and homogenized using a homogenizer (Polytron; manufactured by Kinematica). After centrifugation, each precipitate was suspended in 50 mM Tris-HCl (pH 7.5) containing 10 mM MgCl₂ and used as a CHO membrane fraction or a GPRg2 membrane fraction. The ¹²⁵I-labeled prokineticin 2 ([¹²⁵I]-PK2) was prepared from 5 µg of the prokineticin 2 purified in Example 5 in accordance with the IODO-GEN Iodination Tube (manufactured by Pierce) method using Iodaine-125 (manufactured by Perkin-Elmer).

The [¹²⁵I]-PK2 was added to 30 µg of each of the aforementioned CHO membrane fraction and GPRg2 membrane fraction to a final concentration of 500 pM, incubated at room temperature for 1 hour in 50 µl of a solution comprising 50 mM Tris-HCl (pH 7.5), 10 mM MgCl₂ and 0.1% BSA, and then recovered on a glass filter using a cell harvester. A micro-scintillator was added to the glass filter, and the total amount bonded to the membrane fractions was measured using a liquid scintillation counter. In addition, the amount non-specifically bonded to the membrane fractions was measured by adding 0.5 µM in final concentration of unlabeled prokineticin 1 or 2 (namely the prokineticins purified in Example 5) to the aforementioned test. As a result, it was found that the [¹²⁵I]-PK2 specifically binds to the membrane fraction of the GPRg2-stably expressing CHO cell. On the other hand, the specific binding was not observed in the membrane fraction of CHO cell gene-transferred with the empty vector.

Thus, it was confirmed that the GPRg2 receptor of the present invention is a receptor having binding affinity for prokineticin 2. It became possible to screen a substance having binding affinity for the GPRg2 receptor, by carrying out a binding test using a cell capable of expressing the GPRg2 receptor of the present invention and in the presence of an agent to be tested. Such a substance is a substance having an activity to reinforce the activity of prokineticin 1 or 2 (namely a neuronal death inhibitor) or a substance having an antagonist activity of inhibiting the action of prokineticin 1 or 2 upon GPRg2 (namely a hyperalgesia-treating agent).

### (Example 8) Confirmation of gene expression of GPRg2 in human spinal cord dorsal root ganglion

The spinal cord dorsal root ganglion is a nerve tissue, which undergoes projection of primary perception and carries a role in transmitting a stimulus of pain sensed by a nociceptor to the brain. Thus, an agent capable of reducing excitation of spinal cord nerve cells can inhibit transmission of nocuous stimulus. Such an agent is useful as a pain-treating agent which controls pathological pains such as the pains in rheumatoid arthritis and osteoarthritis. Though there exists a type that does not accompany nocuous stimulus, it is generally considered that the neurogenic pain is induced by enhanced excitation of a nerve cell itself, which related to the pain transmission. Thus an agent capable of reducing excitation of spinal cord nerve cells is effective even in the case of treating neurogenic pain (Sotgiu *et al.*, *Somatosens. Mot. Res.,* 9, 227, 1992; Zhang *et al*., *J. Physiol.,* 84, 798, 2000; Ma and Woolf, Pain, 61, 383, 1995; Sotgiu and Biella, Neurosci. Let., 283, 153, 2000).

In order to analyze tissue expression of human GPRg2 gene in human spinal cord dorsal root ganglion, mRNA was purified from a commercially available human spinal cord dorsal root ganglion-derived total RNA (manufactured by Clontech) using a mRNA purification kit (Oligotex-dT30; manufactured by Takara Shuzo), and then a human spinal cord dorsal root ganglion cDNA was prepared by carrying out reverse transcription reaction using a reagent for reverse transcription reaction use (Super Script; manufactured by Gibco). Using the thus synthesized cDNA as the template, PCR was carried out under the conditions described in Example 2. As a result, a DNA fragment of about 1.2 kbp was amplified, thus confirming that GPRg2 is expressed in human spinal cord dorsal root ganglion. Based on such a result, pain-treating agents, particularly a hyperalgesia-treating agent, can be developed by obtaining a compound capable of regulating the activation mechanism of GPRg2 through its screening.

### Industrial Applicability

A neuronal death inhibitory activity (*Eur. J. Neurosci.,* 2001, 13: 1694 - 702) and a hyperalgesia-inducing activity (*Eur. J. Pharmacol*.,) are known as central nervous system actions of prokineticin, so that a useful substance which can control central nervous system actions, for example, a substance useful as an agent for treating dementia and stroke known as neuronal death-accompanying diseases and/or hyperalgesia, can be conveniently screened by the screening method of the present invention that uses the aforementioned receptor and/or a cell transformed with the aforementioned receptor. According to the screening tool of the present invention, a substance useful as a neuronal death inhibitor and/or a hyperalgesia-treating agent can be screened and evaluated.

In addition, a pharmaceutical composition for neuronal death inhibition and/or hyperalgesia treatment use can be produced by using a substance capable of modifying the activity of the aforementioned receptor, as the active agent that can be obtained by the screening tool of the present invention or the screening method of the present invention, and by making it into a pharmaceutical preparation using a carrier, an excipient and/or other additive agents.

### Sequence Listing Free Text

Description of "Artificial Sequence" is described in the numeric heading <223> in the following Sequence Listing. Illustratively, the nucleotide sequences represented by SEQ ID NOs:3 to 10 of the Sequence Listing are artificially synthesized primer sequences.

Thus, though the present invention has been described in the foregoing by its specific embodiments, modifications and improvements obvious to those skilled in the art are included in the scope of the invention.

## Claims

1. A screening tool for a neuronal death inhibitor, which is a polypeptide consisting of an amino acid sequence of SEQ ID NO:2, or a polypeptide comprising an amino acid sequence represented by SEQ ID NO:2 in which 1 to 10 amino acids therein are deleted, substituted and/or added, wherein
its activity can be verified based on the increase in intracellular calcium concentration in the presence of prokineticin.

2. A screening tool for a neuronal death inhibitor, which is a polypeptide consisting of an amino acid sequence having a 90% or more of homology with an amino acid sequence of SEQ ID NO:2, wherein
its activity can be verified based on the increase in intracellular calcium concentration in the presence of prokineticin.

3. A screening tool for a dementia-treating agent and a stroke-treating agent as neuronal death inhibitors and/or a hyperalgesia-treating agent, which is the polypeptide described in claim 1 or 2.

4. A screening tool for a neuronal death inhibitor, which is a cell expressing the polypeptide described in claim 1 or 2.

5. A screening tool for a dementia-treating agent and a stroke-treating agent as a neuronal death inhibitor and/or a hyperalgesia-treating agent, which is the cell described in claim 4.

6. A method for detecting whether or not a compound to be tested is a prokineticin receptor antagonist or antagonist, comprising the steps of:
allowing the cell according to claim 4 or 5, or a cell membrane thereof to contact with a compound to be tested in the presence of prokineticin ;and
analyzing changes in the activity of the said polypeptide.

7. A method for screening a neuronal death inhibitor,comprising the steps of:
allowing the cell according to claim 4 or 5, or a cell membrane thereof to contact with a compound to be tested in the presence of prokineticin; and
analyzing changes in the activity of the said polypeptide.

8. A method for screening a dementia-treating agent and a stroke-treating agent as neuronal death inhibitors and/or a hyperalgesia-treating agent, comprising the steps of:
allowing the cell according to claim 4 or 5, or a cell membrane thereof to contact with a compound to be tested in the presence of prokineticin; and
analyzing changes in the activity of the said polypeptide.

9. A process for producing a pharmaceutical composition for dementia treatment and stroke treatment, or for neuronal death inhibition, and/or hyperalgesia treatment, comprising the steps of:
carrying out screening using the screening method according to claim 7 or 8; and
producing a pharmaceutical preparation using the substance obtained by the said screening.
